# EUROPEAN PATENT APPLICATION

(11) **EP 2 702 999 A1**
(43) Date of publication of application: **05.03.2014**
(21) Application number: 12777022.0
(22) Date of filing: 25.04.2012
(51) Int. Cl.: A61K 31/711, A61K 31/7125, A61K 47/48, A61K 48/00, A61P 25/00, A61P 25/28, C07K 14/145, C12N 15/113

(54) **BRAIN-TARGETING FUNCTIONAL NUCLEIC ACID AND USE THEREOF**

(30) Priority: 28.04.2011 JP 2011100278
(71) Applicant: National University Corporation Nagoya University, Aichi 464-8601 (JP)
(72) Inventor: SUZUMURA Akio, Nagoya-shi Aichi 464-8601 (JP); MIZUNO Tetsuya, Nagoya-shi Aichi 464-8601 (JP)
(74) Representative: Steinecke, Peter
(86) International application number: PCT/JP2012/061115
(87) International publication number: WO 2012/147805

(57) **Abstract**

The purpose of the invention is to provide a novel therapeutic agent for Alzheimer's disease and use thereof Provided is a therapeutic agent for Alzheimer's disease, which contains a CpG oligodeoxynucleotide structure having a brain migration and improved stability or a salt thereof as an active ingredient.

SEQUENCE LISTING

<110>  NATIONAL UNIVERSITY CORPORATION NAGOYA UNIVERSITY
       SUZUMURA, Akio
       MIZUNO, Tetsuya

<120>  FUNCTIONAL NUCLEOTIDE AND USE THEREOF

<130>  NU11004P

<150>  JP P2011-100278
<151>  2011-04-28

<160>  5

<170>  PatentIn version 3.5

<210>  1
<211>  12
<212>  DNA
<213>  Artificial Sequence

<220>
<223>  CpG olygodeoxynucleotide


<220>
<221>  misc_feature
<222>  (1)..(12)
<223>  phosphorothioated

<400>  1
catgacgttc ct                                                          12


<210>  2
<211>  24
<212>  DNA
<213>  Artificial Sequence

<220>
<223>  CpG olygodeoxynucleotide


<220>
<221>  misc_feature
<222>  (2)..(2)
<223>  phosphorothioated

<220>
<221>  misc_feature
<222>  (5)..(5)
<223>  phosphorothioated

<220>
<221>  misc_feature
<222>  (13)..(13)
<223>  phosphorothioated

<220>
<221>  misc_feature
<222>  (21)..(21)
<223>  phosphorothioated

<400>  2
tcgtcgtttt gtcgttttgt cgtt                                              24


<210>  3
<211>  29
<212>  PRT
<213>  Rabies Virus

<400>  3

Tyr Thr Ile Trp Met Pro Glu Asn Pro Arg Pro Gly Thr Pro Cys Asp
1               5                   10                  15


Ile Phe Thr Asn Ser Arg Gly Lys Arg Ala Ser Asn Gly
            20                  25


<210>  4
<211>  30
<212>  PRT
<213>  Rabies Virus

<400>  4

Tyr Thr Ile Trp Met Pro Glu Asn Pro Arg Pro Gly Thr Pro Cys Asp
1               5                   10                  15


Ile Phe Thr Asn Ser Arg Gly Lys Arg Ala Ser Asn Gly Cys
            20                  25                  30


<210>  5
<211>  20
<212>  DNA
<213>  Artificial Sequence

<220>
<223>  CpG olygodeoxynucleotide

<400>  5
tccatgacgt tcctgatgct                                                   20

## Description

### TECHNICAL FIELD

The present invention relates to an application of functional nucleic acid to treatment for Alzheimer's disease. Specifically, the invention relates to a therapeutic agent for Alzheimer's disease using a functional nucleic acid having a CpG motif and use thereof. This application is based on and claims priority from Japanese Patent Application No. 2011-100278 filed on April 28, 2011, the entire disclosure of which is incorporated by reference herein.

### BACKGROUND ART

The number of patients of Alzheimer's disease increases with a progress to an aging society, but a therapeutic agent approved in this country is presently only an inhibitor for acetylcholine esterase. Novel therapeutic agents have been developed, focusing on Aβ production suppression or Aβ decomposition promotion such as β and γ secretase inhibitors and Aβ vaccines, which targets Aβ that is considered as a pathogenic protein of Alzheimer's disease, but an effective therapeutic method has not been established yet. The neurotoxicity of Aβ is considered to be due to fibrillar Aβ (fAβ) that is deposited in the brain as an insoluble amyloid fiber. However, in recent years, it has been revealed that a soluble Aβ oligomer (oAβ) causes further stronger neurodegeneration. The neurotoxic action thereof is considered to be caused by disturbance of synapse plasticity, oxidative stress by reactive oxygen species (ROS), and disturbance of insulin receptor function in the hippocampus and the like. Accordingly, importance of control of Aβ oligomer is suggested in the treatment strategy of Alzheimer's disease.

Microglia, which are an immune cells in the brain, are shown to be involved in the pathology of Alzheimer's disease. Microglia cluster in the senile plaque, and involved in clearance of not only the aggregated Aβ but also the Aβ oligomer. On the other hand, microglia has an aspect as an inflammatory cell, and the excessive activation thereof has contrary actions such as production of neurotoxic factors such as inflammatory cytokine, ROS and glutamate. In the activation of microglia for the clearance of Aβ, a signal of Toll-like receptor (TLR), which is a receptor associated with activation of the innate immunity, play inevitable roles.

The research group of the present inventors continued researches on the focus of Toll-like receptor 9 (TLR9) that is involved with activation of the innate immunity, and found out that functional nucleic acid CpG oligodeoxynucleotide (CpG-ODN), which is a ligand of TLR9, reinforces Aβ clearance performance of the microglia, which are immune cells in the brain, and induces an anti-oxidation enzyme heme oxygenase 1 (HO-1), whereby to suppress the neurotoxicity of the oligomer Aβ in the co-cultivation of the neurons and the microglia, and further improve the cognitive function and the pathological findings of the Alzheimer's disease model mouse by intraventricular administration of CpG-ODN (Non-Patent Document 1). Researches on the importance of the TLR signal of Alzheimer's disease have also proceeded by other research groups, and as a result thereof, the usefulness of CpG-ODN when administered for several months from the periphery was reported (Non-Patent Document 2). However, there are various problems such that other immune cells are activated and the side reactions easily occur with the periphery administration, and that CpG-ODN is easily decomposed, and hardly migrated into the brain.

### PRIOR ART DOCUMENT

### NON-PATENT DOCUMENT

Non-Patent Document 1: The American Journal of Pathology. 175:2121-2131, 2009
Non-Patent Document 2: The Journal of Neuroscience. 29:1846-1854, 2009

### SUMMARY OF THE INVENTION

### PROBLEM TO BE SOLVED BY THE INVENTION

A functional nucleic acid CpG-ODN, which is a TLR9 ligand, is expected to be applied and clinically applied as a therapeutic agent for Alzheimer's disease. However, as described above, CpG-ODN reported in the past did not pass through blood-brain barrier to enter the brain, and cannot be used in actual treatment. In addition, CpG-ODN has a great room for improvements particularly with respect to stability or the like in consideration of attack of a nuclease when administered to a living body, and the like.

### MEANS FOR SOLVING PROBLEM

In view of the problems described above, the research group of the present inventors have tried optimization of functional nucleic acid CpG-ODN, and tried to enhance migration of CpG-ODN into the brain in order to enable administration from the periphery with a final target of clinical application. At the end of trials and errors, the research group of the present inventors have succeed in synthesis of a novel molecule (referred to as RVG-CpG) that has high stability and exhibits good migration into the brain by performing phosphorothioate modification for newly designed sequences and linking a rabies virus glycoprotein-derived RVG peptide. It has been found out that the molecule keeps performance of Aβ clearance and anti-oxidation enzyme HO-1 induction potency, and suppresses the neurotoxicity of the oligomer Aβ in co-cultivation of neuronal cells and the microglia. Furthermore, the molecule has significantly improved the cognitive function of an Alzheimer's disease model mouse. As described above, it has been supported that the molecule is very effective with respect to Alzheimer's disease, and it has been shown that the technique adopted in synthesis of the molecule (the phosphorothioate modification for the stabilization, and the RVG peptide linkage for imparting brain migration) is effective for clinical application of functional CpG-ODN.

The inventions described below are based on the results described above.
[1] A therapeutic agent for Alzheimer's disease, which comprises a structure in which an oligodeoxynucleotide comprising a CpG motif and being phosphorothioate-modified is linked to a rabies virus glycoprotein-derived RVG peptide, or a pharmacologically acceptable salt thereof.
[2] The therapeutic agent for Alzheimer's disease described in [1], wherein the structure exhibits an action of reinforcing the nerve-protective action of microglia.
[3] The therapeutic agent for Alzheimer's disease described in [1], wherein the action is specific to microglia.
[4] The therapeutic agent for Alzheimer's disease described in any one of [1] to [3], wherein the oligodeoxynucleotide is CpG B class.
[5] The therapeutic agent for Alzheimer's disease described in any one of [1] to [4], wherein the CpG motif consists of gacgtt.
[6] The therapeutic agent for Alzheimer's disease described in any one of [1] to [5], wherein the oligodeoxynucleotide has a structure in which one to several nucleotides are linked to both sides of the CpG motif, respectively.
[7] The therapeutic agent for Alzheimer's disease described in [6], wherein the oligodeoxynucleotide has a length of 10 to 20 nucleotides.
[8] The therapeutic agent for Alzheimer's disease described in [6], wherein the oligodeoxynucleotide has a length of 10 to 14 nucleotides.
[9] The therapeutic agent for Alzheimer's disease described in [6], wherein the oligodeoxynucleotide consists of a sequence of SEQ ID NO: 1.
[10] The therapeutic agent for Alzheimer's disease described in any one of [1] to [9], wherein all nucleotides that constitute the oligonucleotide are phosphorothioate-modified.
[11] The therapeutic agent for Alzheimer's disease described in any one of [1] to [10], wherein the oligodeoxynucleotide and the RVG peptide are linked via a disulfide bond at the position of a cysteine residue in the RVG peptide.
[12] The therapeutic agent for Alzheimer's disease described in any one of [1] to [11], wherein the RVG peptide is linked to the 5' end of the oligodeoxynucleotide.
[13] The therapeutic agent for Alzheimer's disease described in any one of [1] to [12], wherein cysteine is added to the N-terminus or C-terminus of the RVG peptide, and the oligodeoxynucleotide is linked to also at the position of the cysteine.
[14] The therapeutic agent for Alzheimer's disease described in [13], wherein two molecules of the oligodeoxynucleotide and one molecule of the RVG peptide are linked.
[15] The therapeutic agent for Alzheimer's disease described in any one of [1] to [14], wherein the RVG peptide consists of a sequence of SEQ ID NO: 3.
[16] Use of the structure defined in any one of [1] to [15] for manufacture of a therapeutic agent for Alzheimer's disease.
[17] A method of treating Alzheimer's disease, which comprises a step of administering a therapeutically effective amount of the therapeutic agent for Alzheimer's disease described in any one of [1] to [15] to a patient having Alzheimer's disease.
[18] A structure in which a phosphorothioate-modified oligodeoxynucleotide consisting of a sequence of SEQ ID NO: 1, and a rabies virus glycoprotein-derived RVG peptide consisting of a sequence of SEQ ID NO: 3 are linked through a disulfide bond at the position of a cysteine residue in the RVG peptide.
[19] The structure described in [18], wherein cysteine is added to the N-terminus or C-terminus of the RVG peptide, and the oligodeoxynucleotide is linked to also at the position of the cysteine.
[20] The structure described in [18] or [19], wherein all nucleotides that constitute the oligonucleotide are phosphorothioate-modified.

### BRIEF DESCRIPTION OF THE DRAWINGS

Fig. 1 is a schematic diagram that describes the phosphorothioate modification.
Fig. 2 illustrates a linkage between CpG-ODN and rabies virus glycoprotein-derived RVG peptide. In this example, CpG-ODN and the RVG peptide are linked via a linear carbon chain (C6) and a disulfide bond.
Fig. 3 is a graph that illustrates comparison ofmicroglia activation actions of the prepared various CpG-ODNs (results of MTS assay). * represents significant difference from the control (-: no addition).
Fig. 4 is a graph that illustrates comparison of Aβ oligomer reduction effects of the prepared various CpG-ODNs. oAβ represents the Aβ oligomer.
Fig. 5 is a graph that illustrates comparison of neuroprotective actions of the prepared various CpG-ODNs. The survival rate of nerve cells when each CpG-ODN was added was compared with that when no CpG-ODN was added (the second from the left). * represents significant difference from the control (no addition). oAβ represents the Aβ oligomer.
Fig. 6 illustrates the structure in which CpG-ODN and RVG peptide are linked. CpG-ODN is linked to a RVG peptide in which cysteine is added to the C-terminus (RVG-Cys peptide). The linking sites are two spots of the position of cysteine in the center of the RVG-Cys peptide, and the position of cysteine added to the C-terminus, resulting in a structure in which two molecules of CpG-ODN and one molecule of the RVG-Cys peptide are linked (RVG-CpG). With respect to CpG-ODN, only the linkage (5' end) is shown, and the others are ommited.
Fig. 7 illustrates results of fear conditioning learning test using Alzheimer's disease model mouse. RVG-Cys-127-5 was intraperitoneally administered to an Alzheimer's disease model mouse (APP/PS1 transgenic mouse), and the degree of improvement for the cognitive function was investigated. PBS was administered to a control. * represents significant difference from the control (PBS). WT represents a syngeneic wild-type mouse. APP/PS1 represents an APP/PS1 transgenic mouse.
Fig. 8 is a graph illustrating Aβ oligomer reduction effects of RVG-Cys-127-5. * represents significant difference from the control (oAβ: no addition of RVG-Cys-127-5). oAβ represents the Aβ oligomer.
Fig. 9 is a graph illustrating neuroprotective action of RVG-Cys-127-5. Fig. 9 illustrates comparison of the survival rate of nerve cells when RVG-Cys-127-5 was added (right) with that when RVG-Cys-127-5 was not added (center). * represents significant difference from the control (cont: no addition of RVG-Cys-127-5). cont represents the control, and oAβ represents the Aβ oligomer.
Fig. 10 is a graph illustrating anti-oxidative enzyme HO-1 production effects of RVG-Cys-127-5. * represents significant difference from the control (oAβ: no addition of RVG-Cys-127-5). oAβ represents the Aβ oligomer.

### DETAILED DESCRIPTION OF PREFERRED EMBODIMENTS

A first aspect of the invention relates to a therapeutic agent for Alzheimer's disease. The term "therapeutic agent" in the specification refers to a medicine that exhibits therapeutic or preventive effects with respect to Alzheimer's disease, which is a target disease or pathology. The therapeutic effects encompass alleviation (relief) of characteristic symptoms or accompanying symptoms of Alzheimer's disease, inhibition or delay of aggravation of the symptoms, and the like. The latter can be said to be one of preventive effects in terms of preventing aggravation. As described above, therapeutic effects and preventive effects have partially redundant concepts, and it is difficult to clearly distinguish between them, and such distinction has little usefulness. Meanwhile, one of typical preventive effects is inhibition or delay of relapse of characteristic symptoms of Alzheimer's disease. Meanwhile, any substance corresponds to a therapeutic agent for Alzheimer's disease as long as it exhibits any therapeutic effects or preventive effects, or both of them with respect to Alzheimer's disease.

The active ingredient of the invention is a structure in which an oligodeoxynucleotide comprising a CpG motif and being phosphorothioate-modified is linked to a rabies virus glycoprotein-derived RVG peptide, or a pharmacologically acceptable salt thereof. That is to say, the active ingredient in the invention is a molecule of a structure in which a certain oligodeoxynucleotide and a prescribed RVG peptide are linked. The active ingredient of the invention exhibits a characteristic action with respect to microglia, that is, an action of reinforcing the nerve-protective action of microglia. More specifically, the active ingredient of the invention can enhance Aβ oligomer treatment performance of microglia without inducing production of glutamic acid, nitric oxide and TNF-α, which are neuropathic factors. The action is typically specific to microglia. Herein, the term "specific to microglia" means that the active ingredient of the invention has an action to microglia whereas it has no substantial action or significantly low action against other cells (particularly monocytes, macrophages, B cells and dendritic cells in the periphery).

On the other hand, the active ingredient of the invention has an action to microglia, and can promote production of anti-oxidation enzyme heme oxygenase 1 (HO-1). This action is effective for suppressing production of reactive oxygen species (ROS) by the Aβ oligomer.

### (1) CpG oligodeoxynucleotide (CpG-ODN)

The oligodeoxynucleotide in the invention comprises a CpG motif. Herein, the term "motif' refers to a characteristic common sequence having a certain function. The "CpG motif' is an oligoDNA consisting of certain 6 bases (5'-purine-purine-CG-pyrimidine-pyrimidine-3'). In one aspect of the invention, gacgtt is contained as a sequence of the CpG motif moiety.

The oligodeoxynucleotide that comprises the CpG motif can be classified into 3 classes, that is, Class A, Class B and Class C by characteristics of the structure thereof In Class A of CpG-ODN, a phosphodiester bond is used in the CpG motif moiety. Class A of CpG-ODN strongly induces IFN-α secretion of a plasmacyte-like dendritic cell (pDC). On the other hand, Class A of CpG-ODN has nearly no strong maturation action for the plasmacyte-like dendritic cell, and, has low action to B cell. Class B of CpG-ODN is linear, and has a phosphorothioate skeleton. Class B of CpG-ODN is typically completely modified with phosphorothioate. Class B of CpG-ODN generally strongly induces growth of B cell and maturation of the plasmacyte-like dendritic cell (pDC) whereas it has nearly no induction of IFN-α secretion of the plasmacyte-like dendritic cell. Class C has a palindrome structure at 3' end side, and forms a double stranded chain. Class C of CpG-ODN activates B cell and NK cell, and induces IFN-γ.

Class B of CpG-ODN is preferably used in the invention due to the fact that Class B of CpG-ODN exhibits particularly strong nerve-protective action (see Examples described below and The American Journal of Pathology. 175: 2121-2131, 2009). The position and the degree of the phosphorothioate modification (that is, introduction position and introduction number of a phosphorothioate bond) are not limited. The degree of the phosphorothioate modification is desirably high from stabilization of the phosphorothioate-modification site, and improvement of decomposition resistance with respect to nuclease. Class B of CpG-ODN in which all nucleotides constituting CpG-ODN are phosphorothioate-modified is particularly preferably used.

Preferably, CpG-ODN in the invention has a structure in which one to several nucleotides are linked to both sides of the CpG motif, respectively. In other words, the CpG motif is not located at the ends. The number of the nucleotides linked to the 5' side or the 3' side of the CpG motif is not particularly limited, but the molecular weight is desirably not high in consideration of the brain migration. The number of the nucleotides linked to the 5' side of the CpG motif is preferably 1 to 8, more preferably 2 to 6, and further preferably 2 to 4. The number of the nucleotides linked to the 3' side of the CpG motif is similar.

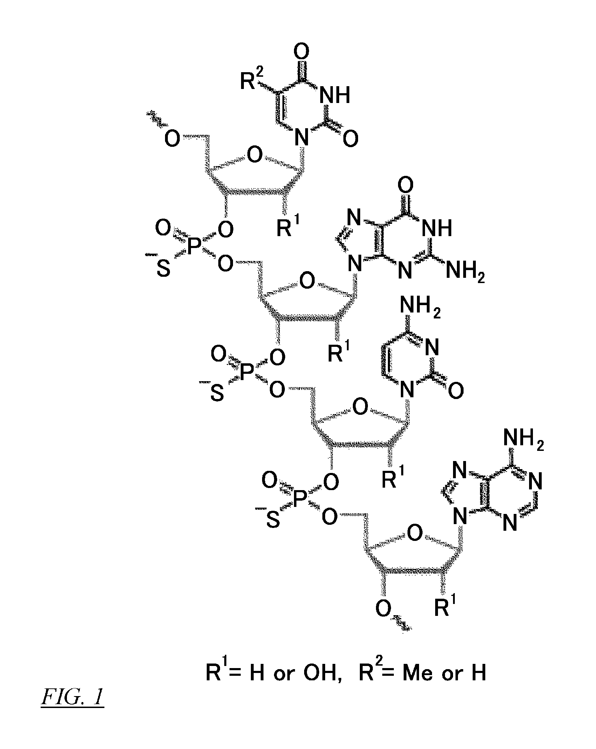

As described above, the molecular weight of CpG-ODN is desirably not high in consideration of the brain migration. Thus, the full length of CpG-ODN is preferably a length of 10 to 25 nucleotides, more preferably a length of 10 to 20 nucleotides, and further more preferably a length of 10 to 14 nucleotides. Specific examples of preferable CpG-ODN in the invention are shown below. Meanwhile, s in the sequences represents the phosphorothioate bond (see Fig. 1).
csastsgsascsgststscscst (SEQ ID NO:1)
tc s gtc s gttttgtc s gttttgtc s gtt (SEQ ID NO: 2)

With respect to the former CpG-ODN (SEQ ID NO: 1), the efficacy was confirmed by experiments using Alzheimer's disease model animals in addition to experiments using cultured cells, and can be said to be particularly preferable CpG-ODN.

The phosphorothioate-modified CpG-ODN may be synthesized with an ordinary method such as a method using solid phase cyanoethylphosphoamidite method and the like (for example, see Journal of the American ChemicalSociety 112:1253, 1990). For isolation and purification of synthesized CpG-ODN, for example, reverse phase high performance liquid chromatography may be used.

### (2) Rabies virus glycoprotein-derived RVG peptide

Migration into the brain is strictly controlled by the blood brain barrier. In the invention, rabies virus glycoprotein-derived RVG peptide is used in order to impart the brain migration. The sequence of the RVG peptide is shown below. In the specification, the peptide is written such that the left end is the amino terminus (N-terminus), and the right end is the carboxy terminus (C-terminus) in accordance with the conventional writing. Note that a delivery system using the rabies virus glycoprotein-derived peptide has been reported (for example, see Nature 448:39-43, 2007; Nature Biotechnology 29:341-345, 2011).
YTIWMPENPRPGTPCDIFTNSRGKRASNG (SEQ ID NO: 3)

Partial alteration or modification of the constituent amino acids of the RVG peptide is accepted as long as the properties required for the invention (that is, imparting of the brain migration) is not harmed. Herein, the "partial alteration" refers to an occurrence of variation (change) in the amino acid sequence by deletion or substitution of one to several (the upper limit is, for example, three, five, or seven) amino acids, addition or insertion of one to several (the upper limit is, for example, three, five, or seven) amino acids, or a combination thereof. In addition, the "modification" refers to partial substitution of a basic structure (typically, a peptide consisting of a sequence of SEQ ID NO: 3) or addition of another molecule or the like, whereby to improve the stabilization or add a new function. One skilled in the art can design a variant such as a substituent using a well-known or common technique. In addition, based on such design, one skilled in the art can easily prepare an intended modification body using a well-known or common technique, and investigate the properties or actions thereof.

Examples of the peptide variant include those obtained by protecting a functional group in a constituent amino acid residue with a suitable protective group (an acyl group, an alkyl group, a monosaccharide, an oligosaccharide, a polysaccharide, and the like); those obtained by adding a sugar chain; various peptide derivatives classified into alkyl amine, alkyl amide, sulfinyl, sulfonylamide, halide, amide, aminoalcohol, ester, aminoaldehyde, and the like in which the N-terminus or the C-terminus is substituted with another atom or the like; and labeled peptides (for example, a peptide labeled with biotin or FITC for the N-terminus, a peptide labeled with a fluorescent dye, and the like). Meanwhile, the protective group is linked by an amide bond, an ester bond, an urethane bond, an urea bond, or the like depending on the peptide site to which the protective group is bonded, or the kind of the protective group to be used, or the like.

The peptides in the invention (the RVG peptide, or an altered form or modified form thereof) can be manufactured using a known peptide synthesis method (for example, a solid phase synthesis method and a liquid phase synthesis method). Meanwhile, the peptides in the invention can be prepared by extracting and purifying from a biomaterial.

The peptides in the invention may be also prepared with a genetic engineering technique. That is to say, the peptides in the invention can be prepared by introducing a nucleic acid that codes the peptides in the invention to a suitable host cell, and recovering a peptide expressed in a transformant. The recovered peptide is purified as necessary. The recovered peptide may be subjected to a suitable substitution reaction, and converted to a desired altered form.

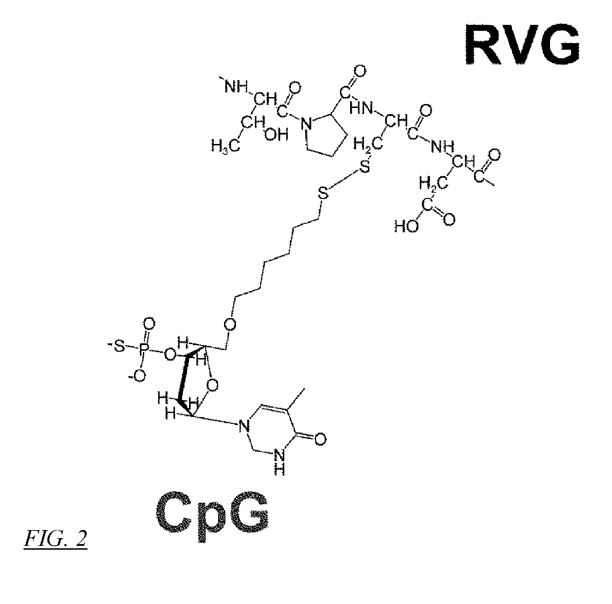

In the structure that is the active ingredient of the invention, CpG-ODN and the RVG peptide are linked. For the linkage, various techniques suitable for a linkage of a nucleic acid and a peptide may be used. In one aspect, CpG-ODN is linked to at the cysteine position in the RVG peptide via a disulfide bond. For such linkage, the method developed by John J. Turner and the others may be used (for example, see Nucleic Acids Research, 33:27-42, 2005). In the method, for example, a thiolation modification agent is used, and as a result thereof, the 5' end side of CpG-ODN and the RVG peptide are linked through a disulfide bond via a carbon chain (see Fig. 2). The kind of the carbon chain (straight chain or branched chain, length) may be changed depending on the modification agent to be used. The carbon number of the carbon chain is, for example, C3 to C10. However, the length of the carbon chain is preferably about C3 to C7 since the structure is preferably low molecular in consideration of the brain migration.

In one embodiment of the invention, cysteine is added to the N-terminus or the C-terminus of the RVG peptide. The cysteine is used for the linkage of CoG-ODN. That is to say, in this embodiment, CpG-ODN is linked to at the position of cysteine at the end in addition to the position of a cysteine residue in the RVG peptide. That is to say, a structure in which two molecules of CpG-ODN and one molecule of the RVG peptide are linked, is used as the active ingredient. In this case, the structures of the two molecules of CpG-ODN are not necessarily the same. However, typically, the same structures of the two molecules of CpG-ODN are used to form the structure. The sequence of the RVG peptide in which cysteine is added to the C-terminus, is shown below.
YTIWMPENPRPGTPCDIFTNSRGKRASNGC (SEQ ID NO: 4)

One to several cysteines may be further added to the N-terminus side or the C-terminus side. The number of cysteines added as described above is not particularly limited, but for example, 1 to 10. A part or all of the added cysteines is subjected to the bonding to CpG-ODN. By this, it is possible to form a structure in which the ratio of the molecule number of CPG-ODN to the molecule number of the RVG peptide has increased (for example, a structure in which one molecule of the RVG peptide is linked to 3 to 10 molecules of CPG-ODN). It is not necessary that cysteine is directly added to the N-terminus side or the C-terminus side of the RVG peptide. The cysteine may be added with other amino acids being interposed. Other amino acids may be interposed between the cysteine residues also when two or more cysteines are added.

A pharmacologically acceptable salt of the structure may be used as the active ingredient of the therapeutic agent of the invention. The "pharmacologically acceptable salt" is, for example, an acid addition salt, a metal salt, an ammonium salt, an organic amine addition salt, or an amino acid addition salt. Examples of the acid addition salt include inorganic acid salts such as a trifluoroacetic acid salt hydrochloric acid salt, a sulfuric acid salt, a nitric acid salt, a phosphoric acid salt, and a hydrogen bromic acid salt; and organic acid salts such as an acetic acid salt, a maleic acid salt, a fumaric acid salt, a citric acid salt, a benzenesulfonic acid salt, a benzoic acid salt, a malic acid salt, an oxalic acid salt, a methanesulfonic acid salt, and a tartaric acid salt. Examples of the metal salt include alkali metal salts such as a sodium salt, a potassium salt, and a lithium salt; alkali earth metal salts such as a magnesium salt and a calcium salt; aluminum salts and zinc salts. Examples of the ammonium salt include salts such as ammonium and tetramethyl ammonium. Examples of the organic amine addition salt include a morpholine addition salt and a piperidine addition salt. Examples of the amino acid addition salt include a glycine addition salt, a phenylalanine addition salt, a lysine addition salt, an aspartic acid addition salt, and a glutamic acid addition salt. Preparations of these salts may be performed by a common technique.

Formulation of the therapeutic agent of the invention may be performed in accordance with an ordinary method. In the formulation, pharmaceutically acceptable other ingredients (for example, a buffer, an excipient, a disintegrator, an emulsifier, a suspending agent, a soothing agent, a stabilizer, a preservative, an antiseptic, saline, a carrier, and the like) may be contained. As the buffer, a phosphate buffer, a citrate buffer, or the like may be used. As the excipient, lactose, starch, sorbitol, D-mannitol, saccharose, or the like may be used. As the disintegrator, starch, carboxy methylcellulose, calcium carbonate, or the like may be used. As the buffer, a phosphate, a citrate, an acetate, or the like may be used. As the emulsifier, gum arabic, sodium alginate, tragacanth, or the like may be used. As the suspending agent, glycerin monostearate, aluminum monostearate, methyl cellulose, carboxymethyl cellulose, hydroxymethyl cellulose, sodium lauryl sulfate, or the like may be used. As the soothing agent, benzyl alcohol, chlorobutanol, sorbitol, or the like may be used. As the stabilizer, propylene glycol, ascorbic acid, or the like may be used. As the preservative, phenol, benzalkonium chloride, benzyl alcohol, chlorobutanol, methyl paraben, or the like may be used. As the antiseptic, benzalkonium chloride, paraoxybenzoic acid, chlorobutanol, or the like may be used.

A dosage form in the formulation is not particularly limited. Examples of the dosage form include an injection, a tablet, a powder, a fine granule, a granule, a capsule, and a syrup.

The therapeutic agent of the invention contains the active ingredient in an amount that is necessary for obtaining expected therapeutic effects (or preventive effects) (that is, therapeutically effective amount). The amount of the active ingredient in the therapeutic agent of the invention generally varies depending on the dosage form, but is set up such that a desired administration amount can be achieved, for example, in a range of about 0.001 weight% to about 95 weight%.

The therapeutic agent of the invention is applied to a subject by oral administration or non-oral administration (intravenous, intraarterial, subcutaneous, intradermal, intramuscular or intraperitoneally injection, percutaneous, pernasal, transmucosal, and the like) depending on the dosage form. These administration routes are not exclusive to each other, and arbitrarily selected two or more routes may be used in combination (for example, oral administration and intravenous injection or the like simultaneously or after the lapse of a prescribed time are performed, etc.). Herein, the "subject" is not particularly limited, and comprises human and mammalian animal besides human (including pet animals, domestic animals, and experiment animals. Specifically, for example, a mouse, a rat, a guinea pig, a hamster, a monkey, a cow, a swine, a goat, a sheep, a dog, a cat, a chicken, a quail, and the like). In a preferable embodiment, the therapeutic agent of the invention is applied to human.

The dosage of the therapeutic agent of the invention is set up such that expected therapeutic effects are obtained. In the set up of the therapeutically effective dosage, the symptoms, the age, the sex, the body weight, and the like of a patient are generally considered. Meanwhile, one skilled in the art can set up a suitable dosage in consideration of these matters. For example, the dosage may be set up such that the amount of the active ingredient is about 10 µg to about 100 µg, preferably about 20 µg to about 50 µg per day for a subject of an adult (about 60 kg of the body weight). As the administration schedule, for example, once to several times per day, once every second day, or once every third day or the like may be adopted. In preparation of the administration schedule, the symptoms of the patient, the effect duration time of the active ingredient, or the like may be considered.

The structure that is the active ingredient of the invention reinforces the nerve-protective action of microglia. Thus, is can be possibly applied to neurodegenerative diseases (for example, Parkinson's disease, amyotrophic lateral sclerosis and Huntington disease) besides Alzheimer's disease, and is useful and has high value as itself. In addition, the structure is also useful as a seed compound in development of a medicine or drug with respect to neurodegenerative diseases including Alzheimer's disease.

### EXAMPLES

The aim was to develop a novel method of treating Alzheimer's disease on the focus of usefulness of CpG-ODN, which is a ligand of Toll-like receptor 9 (TLR9).

### 1. Optimization of CpG-ODN

ODN having a linear structure was designed based on CpG-ODN (CpG subtype B: 5'-TCCATGACGTTCCTGATGCT-3' (SEQ ID NO: 5)) that showed efficacy with respect to Alzheimer's disease. In addition, modification for inhibiting decomposition by nuclease was performed.

### 2. Investigation for efficacy of prepared CpG-ODN

For the prepared CpG-ODN, the efficacy was evaluated with the following method. First, CpG-ODN that activates cultured microglia is selected by MTS assay. On the other hand, the prepared CpG-ODN (1, 10, and 100 nM) under co-cultivation of the neuron and the microglia is administered, and then 5 µM Aβ oligomer is added, and nerve cell death is detected and evaluated after 24 hours. Those exhibiting 70% or more of the survival rate with immunostaining are selected. Then, the selected CpG-ODN is added to the cultured microglia, and then 5 µM Aβ oligomer is added, and the Aβ oligomer is quantitatively determined by ELISA method after 24 hours, and those exhibiting 40% or more reduction are selected.

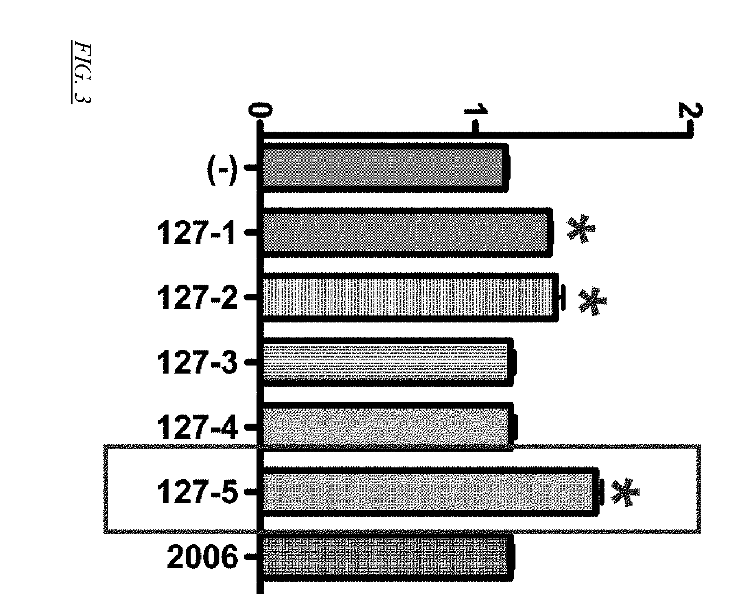

The results of the MTS assay investigation are shown in Fig. 3. The results of the 6 kinds of CpG-ODN (127-1, 127-2, 127-3, 127-4, and 127-5 2006) were compared. 127-1, 127-2, and 127-5 exhibited high activation performance. 127-5 that has been judged to exhibit particularly high efficacy is phosphorothioate-modified for its entirety. Partial phosphorothioate modification is also performed for 127-2. The sequences of these two CpG-ODNs are shown below. S in the sequences represents the phosphorothioate bond.
127-5:csastsgsascsgststscscst (SEQ ID NO: 1)
127-2: tc s gtc s gttttgtc s gttttgtc s gtt (SEQ ID NO: 2)

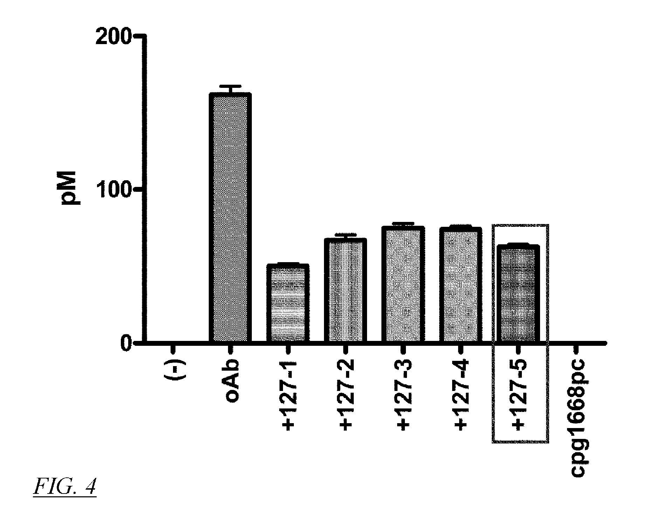

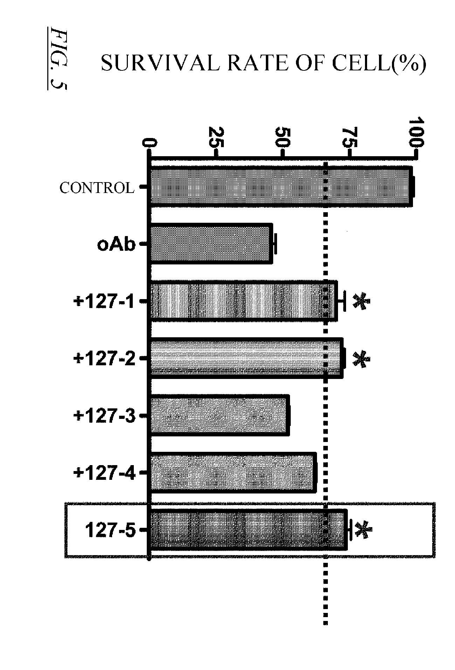

The Aβ oligomer reduction effects of respective CpG-ODNs (results of ELISA method) are shown in Fig. 4. In addition, the effects with respect to the nerve cell death (evaluation for the survival rate by immunostaining) are shown in Fig. 5. It is understood that 127-1, 127-2, and 127-5 effectively reduce the Aβ oligomer, and exhibit the nerve-protective action.

### 3. Preparation of brain-migrating CpG-ODN (addition of brain-migrating peptide)

CpG-ODNs (127-1, 127-2, 127-4, and 127-5), for which efficacy was confirmed, were added with rabies virus glycoprotein-derived RVG peptide (YTIWMPENPRPGTPCDIFTNSRGKRASNG (SEQ ID NO: 3)) to impart brain migration. First, brain-migrating RVG-Cys peptide (YTIWMPENPRPGTPCDIFTNSRGKRASNGC (SEQ ID NO: 4)) in which cysteine (Cys) was added to the C-terminus of the RVG peptide, was prepared. Then, the cysteine residue of the RVG-Cys peptide was converted to pyridyl, and then mixed with 5'-thiolated CpG-ODN under a suitable concentration condition to perform the substitution reaction. The obtained reactant was purified with reverse phase HPCL method.

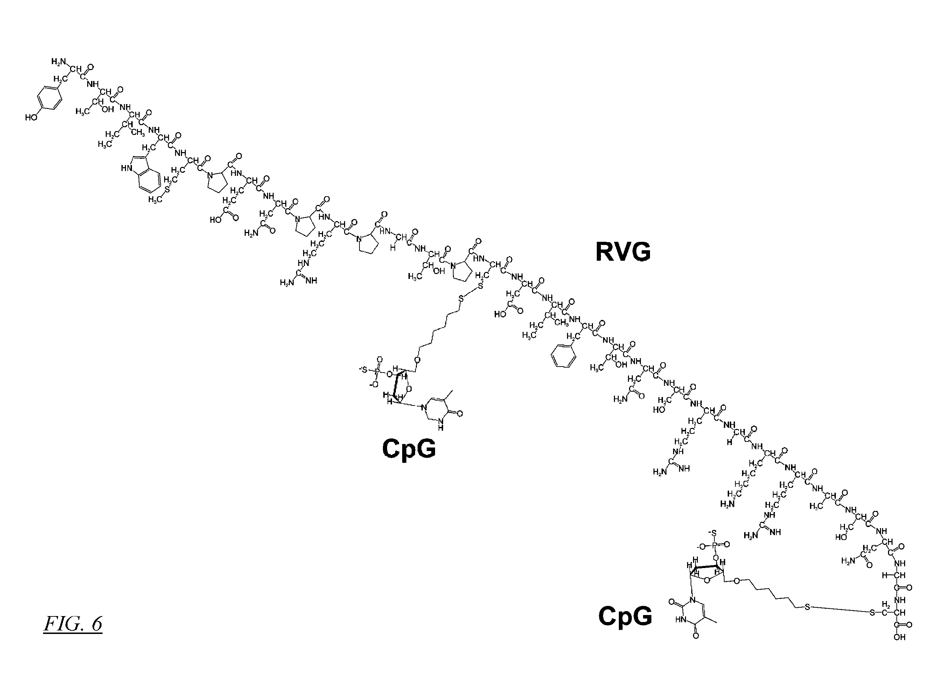

By the operations described above, a structure (RVG-CpG) in which CpG-ODNs are linked via a carbon chain and a disulfide bond to 2 spots of the cysteine positions in the RVG-Cys peptide was obtained(Fig. 6).

### 4. Verification for efficacy of brain-migrating CpG-ODN (RVG-CpG) (Investigation with Alzheimer's disease model mouse)

The therapeutic effect of the brain-migrating CpG-ODN (RVG-Cys-127-5), which was manufactured using 127-5 judged to have the highest efficacy by the investigation at "2." above, was investigated. The fear conditioning learning test (associative learning) was adopted using an Alzheimer's disease model mouse (APP/PS1 transgenic mouse (The Jackson Laboratory)) in the investigation. The test method is briefly described below. RVG-Cys-127-5 was intraperitoneally administered in 1 µg of the dosage every other day per time (three times in total) to an Alzheimer's disease model mouse. On the second day after completion of the total administration, the mouse was put into a test box, and the conditioning was performed by giving electrical stimulation only or electrical stimulation and sonic stimulation. The next day, the freezing behavior was evaluated and quantitatively determined for the case where the mouse was only caged in the box, and for the case where the mouse was further applied with sonic stimulation. It was found out that the freezing behavior was reduced in the Alzheimer's disease model mouse, whereas the freezing behavior significantly increased, and the cognition function improved in the RVG-Cys-127-5 administration group. On the other hand, investigation using cultured cells was also performed in accordance with the evaluation method of "2." above. In addition, promotion action for production of the oxidation enzyme HO-1 in the presence of the Aβ oligomer was also investigated by the method below. RVG-Cys-127-5 was administered to cultured microglia in the presence of the Aβ oligomer, and after 24 hours, the cells were crushed and the HO-1 protein was quantitatively determined using an HO-1 ELISA kit.

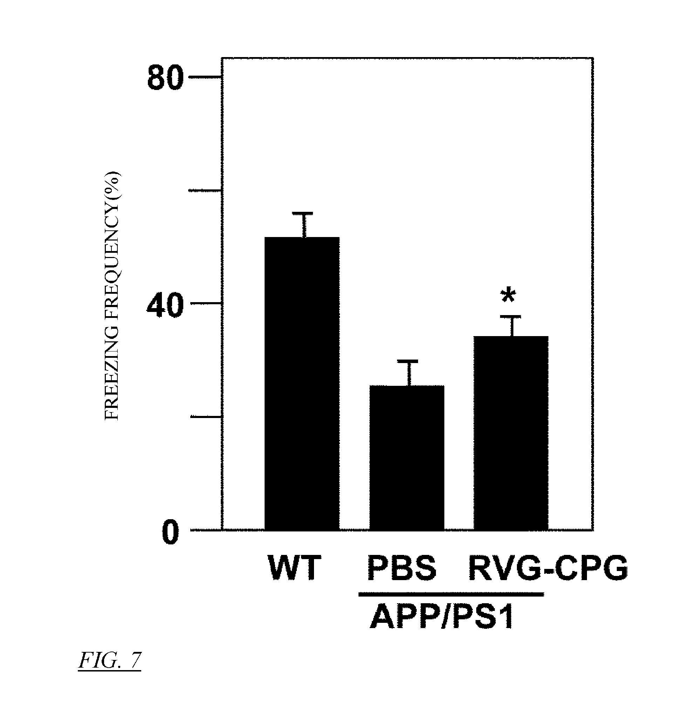

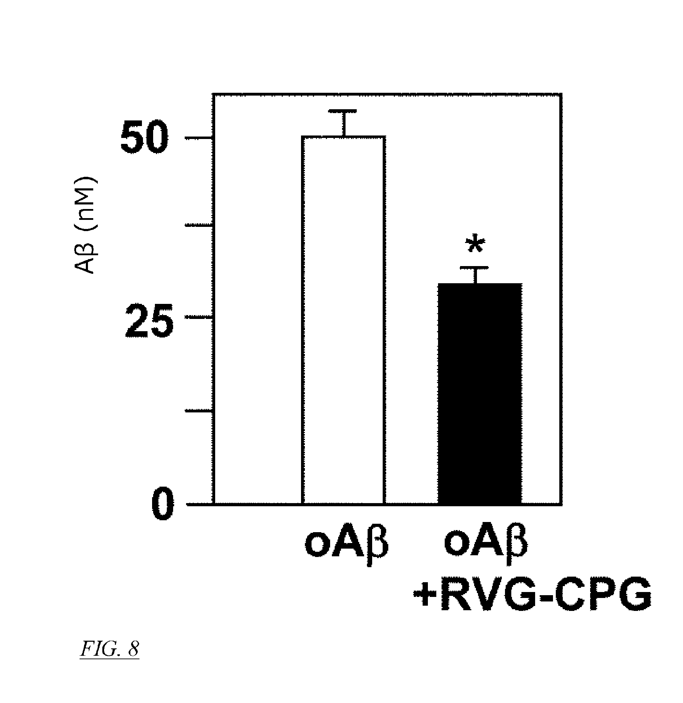

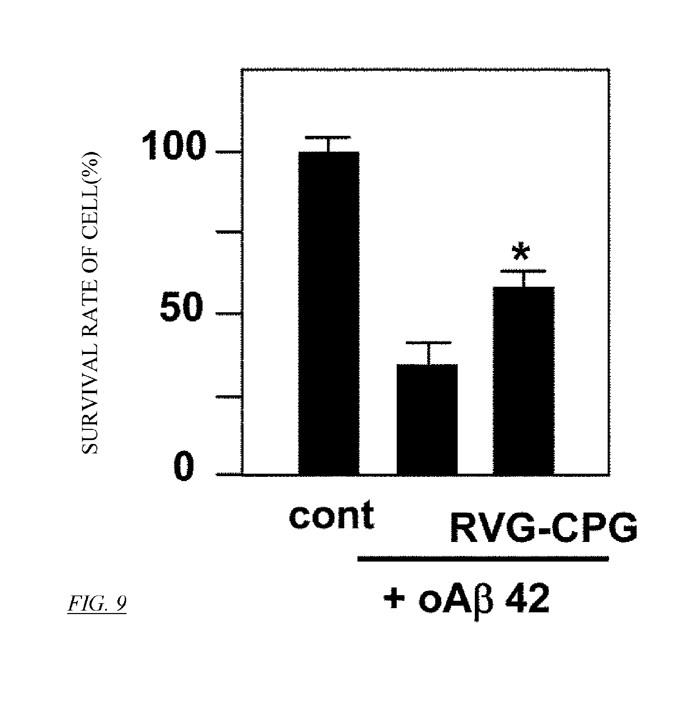

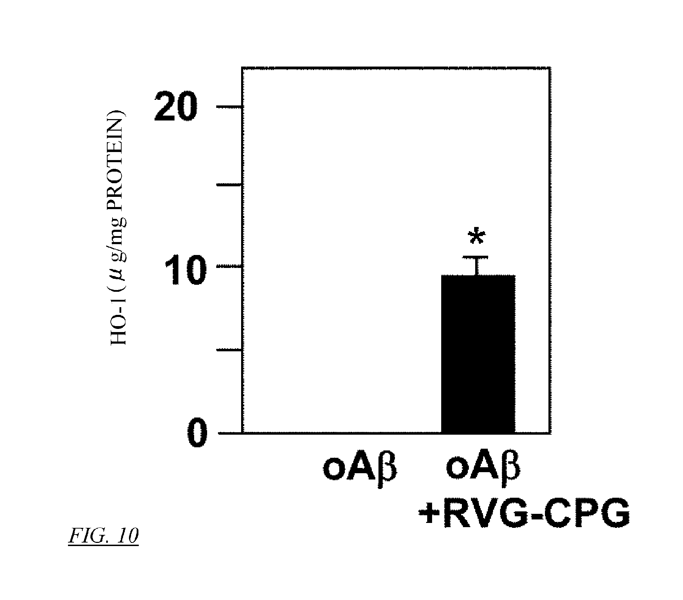

The results of the fear conditioning learning test are shown in Fig. 7. The cognition function disorder was significantly improved by administration of RVG-Cys-127-5. In the experiment using the cultured cells, RVG-Cys-127-5 significantly reduced the amount of the Aβ oligomer (Fig. 8), significantly enhanced the survival rate of nerve cells (Fig. 9), and further promoted production of HO-1 that acts on the nerve protection (Fig. 10). From the results above, it is shown that RVG-Cys-127-5 is effective for Alzheimer's disease.

### 5. Summary

A novel molecule that is excellent in the brain migration and the stability, that is, functional nucleic acid (RVG-CpG), was successfully synthesized. The molecule exhibited nerve-protective action, and significantly improved cognition performance of an Alzheimer's disease model animal with peripheral administration. This fact shows that the molecule is effective for Alzheimer's disease. In addition, possibility of application to other neurodegenerative diseases is strongly suggested. Based on the molecule, development of a novel drug aimed to further improve the drug efficacy is also expected.

On the other hand, the experiment results described above also support the efficacy of the technique that was adopted in the synthesis of the novel molecule (phosphorothioate modification for stabilization, and linkage to the RVG peptide for imparting brain migration). As application of the RVG peptide, famous one is the example of Kumar and the other, in which nine lysines are added to the RVG peptide, bonded electrically to siRNA, and administered (Nature 448:39-43, 2007). In contrast, in the novel molecule successfully synthesized this time, the peptide and the nucleic acid are linked using an S-S bond, and the novel molecule has high stability, and has low synthesis cost. These characteristics are important in practical use.

### INDUSTRIAL APPLICABILITY

The novel RVG-CpG structure, which is the active ingredient of the invention, is excellent in the brain migration and the stability, and exerts drug efficacy by reinforcing the nerve-protective action of the microglia. The novel RVG-CpG structure is greatly expected to be applied to not only Alzheimer's disease, but also other neurodegenerative diseases (for example, Parkinson's disease, amyotrophic lateral sclerosis and Huntington disease). Use of the structure as a seed compound for developing a medicine or drug with respect to neurodegenerative diseases including Alzheimer's disease is also contemplated.

The invention will not be limited to the description of the embodiments and examples of the invention. Various modifications readily made by those skilled in the art are also included in the invention, without departing from the scope of claims. The contents of the articles, unexamined patent publications, and patent applications specified herein are incorporated herein by reference in its entirety.

### SEQUENCE LISTING FREE TEXT

SEQ ID NO: 1: Description of artificial sequence: CpG oligodeoxynucleotide
SEQ ID NO: 2: Description of artificial sequence: CpG oligodeoxynucleotide
SEQ ID NO: 5: Description of artificial sequence: CpG oligodeoxynucleotide

## Claims

1. A therapeutic agent for Alzheimer's disease, which comprises a structure in which an oligodeoxynucleotide comprising a CpG motif and being phosphorothioate-modified is linked to a rabies virus glycoprotein-derived RVG peptide, or a pharmacologically acceptable salt thereof.

2. The therapeutic agent for Alzheimer's disease according to claim 1, wherein the structure exhibits an action of reinforcing the nerve-protective action of microglia.

3. The therapeutic agent for Alzheimer's disease according to claim 2, wherein the action is specific to microglia.

4. The therapeutic agent for Alzheimer's disease according to any one of claims 1 to 3, wherein the oligodeoxynucleotide is CpG B class.

5. The therapeutic agent for Alzheimer's disease according to any one of claims 1 to 4, wherein the CpG motif consists of gacgtt.

6. The therapeutic agent for Alzheimer's disease according to any one of claims 1 to 5, wherein the oligodeoxynucleotide has a structure in which one to several nucleotides are linked to both sides of the CpG motif, respectively.

7. The therapeutic agent for Alzheimer's disease according to claim 6, wherein the oligodeoxynucleotide has a length of 10 to 20 nucleotides.

8. The therapeutic agent for Alzheimer's disease according to claim 6, wherein the oligodeoxynucleotide has a length of 10 to 14 nucleotides.

9. The therapeutic agent for Alzheimer's disease according to claim 6, wherein the oligodeoxynucleotide consists of a sequence of SEQ ID NO: 1.

10. The therapeutic agent for Alzheimer's disease according to any one of claims 1 to 9, wherein all nucleotides that constitute the oligonucleotide are phosphorothioate-modified.

11. The therapeutic agent for Alzheimer's disease according to any one of claims 1 to 10, wherein the oligodeoxynucleotide and the RVG peptide are linked via a disulfide bond at the position of a cysteine residue in the RVG peptide.

12. The therapeutic agent for Alzheimer's disease according to any one of claims 1 to 11, wherein the RVG peptide is linked to the 5' end of the oligodeoxynucleotide.

13. The therapeutic agent for Alzheimer's disease according to any one of claims 1 to 12, wherein cysteine is added to the N-terminus or C-terminus of the RVG peptide, and the oligodeoxynucleotide is linked to also at the position of the cysteine.

14. The therapeutic agent for Alzheimer's disease according to claim 13, wherein two molecules of the oligodeoxynucleotide and one molecule of the RVG peptide are linked.

15. The therapeutic agent for Alzheimer's disease according to any one of claims 1 to 14, wherein the RVG peptide consists of a sequence of SEQ ID NO: 3.

16. Use of the structure defined in any one of claims 1 to 15 for manufacture of a therapeutic agent for Alzheimer's disease.

17. A method of treating Alzheimer's disease, which comprises a step of administering a therapeutically effective amount of the therapeutic agent for Alzheimer's disease according to any one of claims 1 to 15 to a patient having Alzheimer's disease.

18. A structure in which a phosphorothioate-modified oligodeoxynucleotide consisting of a sequence of SEQ ID NO: 1, and a rabies virus glycoprotein-derived RVG peptide consisting of a sequence of SEQ ID NO: 3 are linked through a disulfide bond at the position of a cysteine residue in the RVG peptide.

19. The structure according to claim 18, wherein cysteine is added to the N-terminus or C-terminus of the RVG peptide, and the oligodeoxynucleotide is linked to also at the position of the cysteine.

20. The structure according to claim 18 or 19, wherein all nucleotides that constitute the oligonucleotide are phosphorothioate-modified.
